# EUROPEAN PATENT SPECIFICATION

(11) **EP 3 124 030 B1**
(45) Date of publication and mention of the grant of the patent: **16.03.2022**
(21) Application number: 15386024.2
(22) Date of filing: 30.07.2015
(51) Int. Cl.: A61K 35/583, A61K 35/618, A61K 8/98, A61P 17/02, A61Q 19/00, A61Q 19/08

(54) **METHOD FOR PRODUCING A SNAIL EXTRACT FROM CORNU ASPERSUM AND ITS EXTRACT**
VERFAHREN ZUR HERSTELLUNG EINES SCHNECKENEXTRAKTS AUS CORNU ASPERSUM UND DEREN EXTRAKT
PROCÉDÉ DE PRODUCTION D'UN EXTRAIT D'ESCARGOT PETIT-GRIS (CORNU ASPERSUM) ET LEDIT EXTRAIT

(43) Date of publication of application: 01.02.2017
(73) Proprietor: Aristotle University Of Thessaloniki-Research Committee, 54636 Thessaloniki (GR); Pavlidis, Savvas, 57500 Mesimeri Thessaloniki (GR); Wehrheim, Anne-Gailar, 57500 Mesimeri Thessaloniki (GR)
(72) Inventor: Staikou, Alexandra, GR-54124 Thessaloniki (GR); Garefalaki, Marina-Elena, GR-54249 Thessaloniki (GR)
(74) Representative: Petsis, Christos

(56) References cited:
- WO-A1-2013/011371
- WO-A2-2009/002982
- DE-A1- 1 813 154
- US-A- 5 538 740
- US-A1- 2013 309 296

## Description

### Field of the invention

The present invention relates to a method for producing a snail extract from a selected member of the gastropod family, particularly *Cornu aspersum.*

### Background of the invention

Snails have always been considered as a natural source having numerous therapeutic and biological properties. Land snails have been used in medicine since antiquity and were introduced by Hippocrates, Pliny, and Galen for their medical use. Snails were prepared according to several formulations for internal or external use. Boiled, grilled or crushed with their shell and heated with wine, they appeared curative against several diseases such as vertigo, fainting fits, anthrax and for the spasms of spitting blood accompanying tuberculosis, and for the urine ardor of nephritis. External use of snail extracts was considered to treat pain related to burns, abscesses and other wounds. Thus, eating or drinking snails, or applying them to wounds provided a very effective treatment based on natural products.

The 19^{th} century revealed new insights in the pharmaceutical and medical use of snails with more advanced snail preparations, such as tablets, syrup, snail paste and snail chocolate. They were applied orally and were indicated for resistant colds, tuberculosis and against most inflammations. They were especially indicated against lung diseases and infections, against pathological disorders of the stomach, and some skin diseases *(Baron-Barthelemy 1855*, *Bonnemain 2003).*

The interest in therapeutic and biological properties of snail extract continued in the 20^{th} century when more than 30 enzymes within the digestive mucus were identified, and several enzymes in the pancreostomach, the muscle and lymphatic fluid, had demonstrated mucolytic and spasmolytic activity for patients with chronic bronchitis *(Quevauviller et al 1953, Bonnemain 2003).* Current concern on snail research includes a more detailed approach and has recognized E₂ prostaglandin as a broncho-relaxant substance *(Pons et al 1999*), a lectin from *Helix pomatia* as a prognostic factor for several cancers *(Dwek et al 2001, Brooks & Leathern 1991)*, whereas hundreds of neurotoxins derived from sea snails have been evaluated by the FDA for their powerful anesthetic effect *(Webster et al 2001).* Interestingly, the molecular basis of the regenerative properties of *Helix aspersa* secretion has been recently identified as it exhibited antioxidant SOD and GST activities *(Brieva et al 2008).* Moreover, an up to date investigation revealed the regenerative properties of the secretion of *Helix aspersa* as it also promotes proliferation, migration and survival of keratinocytes and dermal fibroblasts *in vitro* (*Iglesias-de la Cruz et al 2012).* The topical application of a cream based on snail extract of *Helix aspersa* on dermal burns demonstrated that *Helix aspersa* extract is a natural, safe and effective alternative treatment in open wound management by accelerating skin regeneration and decreasing pain *(Tsoutsos et al 2009).*

Life-cycle and biology of the *Cornu aspersum* also play an important role. *Cornu aspersum,* also known as *Helix aspersa, Cantareus aspersus* and *Cryptomphalus aspersus*, is a terrestrial pulmonate gastropod with as common name 'garden snail'. It is native to the Mediterranean area but has been spread by humans to numerous areas all over the world and is therefore considered as an anthropochorus species. The garden snail is herbivore: it feeds on plants only, notably on numerous types of fruit trees, vegetable crops, garden flowers and cereals. In addition to humans, it is a food source for many animals. Adult snails bear a hard, thin calcareous shell with 4 or 5 whorls. The body of the snail is retracted entirely into the shell when the animal is inactive or threatened, while during times of activity the head and foot emerge. The head bears four tentacles, which can be retracted into the head. The mouth is located beneath the tentacles and contains a chitinous radula which the snail uses to scrape and manipulate food particles. The aperture of the shell is sealed with a thin membrane of dried mucus known as an epiphragm, which helps the snail retain moisture under stressful condition of humidity and temperature. Thermal death by overheating poses a danger for these animals in hot regions. Thus restricting activity to favorable time periods with respect to temperature and humidity can be seen as a further behavioral adaptation of terrestrial snails (*Staikou 1999*). In addition, many land snail species use summer aestivation during times of disadvantageous conditions in order to save water and energy *(Herreid 1977; Barnhart & McMachon 1987; Withers, Pedler & Guppy 1997).* Hibernation represents the inactive phase of snails during the winter due to low temperatures. When hibernating, *Cornu aspersum* avoids ice formation by altering the osmotic components of its blood (or haemolymph) and can survive temperatures as low as -5°C (*Ansart e.a.2002).* During aestivation, the mantle collar has the unique ability to change its permeability to water *(Machin, 1966)*. In combination with an osmoregulatory mechanism similar to that seen during hibernation, this allows *Cornu aspersum* to survive several months of aestivation.

*Cornu aspersum* is a land snail with diverse scientific interest. The biology and behavior of this snail has many applications in biological research and it is used as model organism in numerous fields of biology, including ethology, neurobiology, and reproductive biology. Interestingly, due to the fact that it is an edible land snail, farming of this species has gained attention the last decades notably in the Mediterranean. Therefore, understanding the reproductive cycle and all aspects of the reproductive biology of *Cornu aspersum* is a prerequisite in order to organize a successful snail farm. *Cornu aspersum* is a simultaneous reciprocal and obligatory outcrossing hermaphrodite with internal fertilization, which means that each individual acts in both sexual roles during copulation, by exchanging sperm, whereas self-fertilization never occurs. *Cornu aspersum* mates repeatedly with different partners, i.e. multiple mating, in the course of a reproductive season. It has been reported to mate on average three times up to maximum seven times, (*Fearnley 1993, 1996; Madec & Daguzan 1991, 1993*). *Cornu aspersum* from Greek populations mated on average three times up to maximum seven times, with at maximum five different partners, during the reproductive period *(Koemtzopoulos 2007, Garefalaki 2010).* Furthermore, mating frequency was consistent in F₁ generation snails reared in the lab *(Koemtzopoulos & Staikou, 2007).* Interestingly, multiple mating leading to multiple paternity of the offspring has been related to behavioral -mating order- and anatomical -epiphallus length-reproductive traits in this species *(Garefalaki et al. 2010*)*.* In the field, snails aestivate during the dry period characterizing the Mediterranean climate ranging from June to September. With the first autumnal rainfalls starting from early October, they emerge and immediately start mating. The reproductive period lasts for 2 months. Mating in this species includes elaborate courtship behaviour with optional dart shooting *(Chung, 1987; Adamo & Chase 1988)*. The female part of the reproductive system includes a sperm digesting -bursa copulatrix- and a sperm storing [fertilization pouch-spermatheca complex (FPSC), Tompa, 1984] organ. *Cornu aspersum* copulates for variable time periods ranging from 2 to 12 h *(Koemtzopoulos & Staikou 2007)* during which each snail transfers a spermatophore containing from 10⁶ to 14 X 10¹ *(Rogers & Chase 2001)* sperm into its partner. The spermatophore is formed in the epiphallus and flagellum which are the male parts of the reproductive system. The majority of sperm transferred is digested in the bursa copulatrix, whereas only a small portion of about 0,025% escapes and travels up the spermoviduct to reach the tubules of the spermatheca *(Rogers & Chase 2001)* where it is stored until fertilization. The fertilization of the ova generally occurs several hours or days after mating *(Madec et al 1998)*. The albumen gland provides numerous nutrient substances to the fertilized eggs such as proteins, lipids, polysaccharides. After about two weeks, approximately 80 spherical pearly-white eggs are laid into crevices in the topsoil. The young snails take one to two years to reach maturity.

Snails produce mucus which is crucial in defense and locomotion of molluscs that are exposed to the external environment *(Denny 1980, Davies & Hawkins 1998).* Pedal mucus is composed of a variety of molecules in an aqueous carrier medium, including mainly charged mucopolysaccharides, glycoproteins, proteins, uronic acid, sialic acid, hexosamine, glycosaminoglycans (GAGS), lectins, and proteoglycans *(Skingsley et al 2000).* It is produced from a series of glands used to aid locomotion of the animal over a variety of terrestrial and aquatic terrain. Stimulation of mucus secretion is based on sensation of mechano- and chemo-active changes and predative threat through feedback from sensory array integration by the central nervous system (CNS) *(Deyrup-Olsen 1996 et al 1992)*. Release of pedal mucus from the pedal gland is in the form of membrane encased mucus granules which are ruptured by various cues including mechanical shearing forces. Molluscs produce 60-400 µg of dry mucus/hour/g body weight which has a calorific value of about 24 J/mg dry weight *(Schlichter 1981, Calow 1978)*. This mucus conveys information about the animal's sexual state and direction of locomotion to conspecifics and predators (*Denny 1989)*. The uniqueness of an individual mollusc's mucus at species level has been supported by findings using SDS-PAGE electrophoresis -seven snail species-, which showed that there were few similarities between the individual species' carbohydrate and protein profiles *(Cottrell et al 1993)*.

Other glandular secretions of snails are strongly related to reproduction such as mucus that derives from a pair of digitiform glands and covers the love dart. Dart shot during copulation acts as a hypodermic device to deliver the mucus to the interior of the recipient *(Koene & Chase 1998).* The mucus contains a biologically active substance that could influence the female organs involved in the processing of foreign sperm. Thus, the successful dart shooter may increase the chance that his sperm will fertilize eggs.

Another gland of the reproductive system is the albumen gland which provides numerous nutrients to the fertilized ova before oviposition *(Tompa 1984)*. The albumen gland of land snails is the organ that fluctuates most in size during different stages of the reproductive cycle; it is largest immediately before egg laying, when it can be 6% of the total body weight *(Runham & Laryea 1968) and* may contain more than 85% of the total body polysaccharides, resp. 100% of the galactogen. Immediately after egg laying, the size of this organ is reduced. Apart from its role in galactogen synthesis and nutrient enrichment of the ova, it has been proposed to contain antimicrobial substances (*Renwrantz & Berliner 1978).* In *Cornu aspersum* the fertilization pouch-spermatheca complex (FPSC) is an organ of the reproductive system embedded in the albumen gland, playing a fundamental role in sperm storage (allosperm) and fertilization of the ova. As sperm is stored for prolonged periods up to over a year, the FPSC must contain factors that promote cell viability, whereas growth factors inducing cell division may also be present in this organ for the fertilized ova. A recent study revealed the potential effect of spermatheca gland extract of a marine snail on wound healing in an animal model *(Kumar et al 2008).*

The digestive tract and the digestive gland is composed of calcium, excretory and digestive cells, which absorb and secrete food material, store glycogen and fat, and play a role in shell regeneration *(Sumner 1965).* In addition, snail products thus provide skin regeneration. Skin regeneration due to ageing, environmental factors, such as UV light exposure, or injury is a complex procedure which involves alterations in the remodeling process of the extracellular matrix (ECM), proliferation, migration and survival of new cells, a task carried out by dermal fibroblasts and keratinocytes *(Ashcroft et al 1995).* Proliferation and migration of fibroblasts is activated and ECM components such as elastin, collagen and fibronectin are secreted to renew the elasticity and consistency of the skin. In healthy skin, physiological regenerative events include proliferation and migration of keratinocytes, which is a layer of cells attached to a carpet of specialized extracellular matrix, the basal lamina. The establishment of new adhesions cell-cell and cell-substrate (basal lamina), includes interactions between protein molecules such as E-cadherin (ECD), *β*-catenin, integrin, laminin (basal lamina), hemidesmosomes and intermediate filaments of the cytoskeleton (cytokeratins). E-cadherin (ECD) is a transmembrane protein that mediates intercellular adhesion through other intracellular anchor proteins such as *β*-catenin in adhesion junctions *(Knudsen & Soler 2000).* Hemidesmosomes anchor to the laminin in the basal lamina through specific integrins belonging to the *β*1-subfamiiy for the cell-substrate junctions *(Yamada et al 1995),* while cell-cell adhesions through hemidesmosomes include interactions with the ECM and intermediate filaments of the cytoskeleton (cytokeratins). These interactions are important not only for the integrity of the skin but also for signal transduction leading to cell migration and proliferation, e.g. phosphorylation of the Tyr kinase focal adhesion kinase (FAK) *(Yamada et al 1995, Zouq et al 2009).* Finally, in terms of survival signals, elevated levels of *β*-catenin have also been associated with improved cell survival, as it is incorporated to the transcription of genes related to cell proliferation *(Fuchs et al 2005).*

### Prior Art

The interest in skin regeneration properties of numerous natural substances obtained either from plants or animals including snails, has increased the last few years. Snails secrete large amounts of mucous substances as a defensive response in order to protect themselves from harmful conditions such as radiation, infections, mechanical stress, increased salinity, temperature, sound vibrations, hyperbaric pressure, and hypoxia *(Dittbrenner et al 2009, Kuang et al 2002).* Moreover, snails perform a relatively rapid reconstitution of their broken shell, whereas they never suffer from skin infections or cancer, despite the exposure of their skin to damaging factors such as UV light. The main mechanism of UV induced photo-ageing is a marked increase in the production of reactive oxygen species (ROS) which causes photooxidative damage and decreases cell migration and proliferation. Interestingly, the secretion from *Cornu aspersum* has been proven to have antioxidant activity *(Brieva et al 1999)* and to induce skin regeneration after wound-healing impairment from acute radiodermatitis *(Ledo et al* 1999). In this regard, the secretion of some snails has proven beneficial in the regeneration of burnt skin *(Badiu et al 2008)* and in the management of open wounds *(Tsoutsos et al 2009).* Furthermore, a recent study demonstrated that the secretion from *Cornu aspersum* stimulates fibroblast proliferation, rearrangement of the actin cytoskeleton and stimulates ECM assembly *(Brieva et al 2008).* Additionally, a more recent study revealed the effect of this secretion on proliferation, migration and survival of keratinocytes and dermal fibroblasts *in vitro (Iglesias-de la Cruz et al 2012).* Therefore, the attention of many pharmaceutical companies has been directed, through extensive research programs, on identifying natural products that stimulate skin regeneration using the secretion of snails as a possible treatment of skin diseases.

Until now, the most commonly used method in snail extract acquisition consists of two steps, a first centrifugation of the snails in order to obtain the extract and a further centrifugation of the snail extract as disclosed in patent US 5538740. According to this method, the technique used to obtain the extract from the live gastropod does not change the biological structure of the derived product. Physical stimulus of the snail was induced by centrifugation. Before the physical stimulation, snails were fasted for 5 days to ensure the absence of any toxins present in the secreted fluid. Preferably, a force of about 2000 rpm was used for about 1 minute at 20°C. This procedure was repeated twice, which has the drawback to possibly result in snail mortality. The snails, which were still alive, were removed and the snail extract was obtained, yet at the expense of snail mortality. Snail extract obtained in this way was further processed, on a second step, by centrifugation at 5000 rpm for about 10 min. The resulting supernatant was the final extract acquired.

Said US patent yet deals with both Helix and non-Helix forms which are suitable to obtain glandular secretions, indicating a positive effect in case of infections of the organ. It is stated that preferred gastropoda include a number of 21 species within the genus Helix. However, *Helix aspersa* (*Cornu aspersum*) is not mentioned at all therein, whereas it refers to centrifuging a live "Helix gastropod".

The method disclosed by WO2013/011371 A1 includes a dehydration step during which the aimed mucus is produced.

This document discloses a process and an apparatus for the extraction of snail slime, wherein the procedure for the extraction of burr or slime from snails is to spray a solution of water and produce burr that drips through the permeable surface and is collected in containers.

Finally, DE-1813154 yet discloses a product of snail extract acquisition, wherein the snails are nourished and kept in movement in order to obtain a snail extract as a result of snail crawling.

Skin cream is then prepared from the dried secretions obtained from the snails.

### Aim of the invention

The aim of this invention is to propose an innovative method for acquisition and further processing of snail extract, wherein an attempt is made to develop an alternative method for both steps of the already used process of snail extract acquisition evoked above, on the one hand, in order to avoid both snail centrifugation (first step), which has the drawback to cause snail death, and snail extract centrifugation (second step) during which potential bioactive substances may remain in the residue, which is an additional drawback.

A further object of the invention is to evaluate an improved technique thereby targeting the optimum one, that can be applied in a large scale, at industrial level, such as in cooperation with snail farms, wherein the effect of the bioactive substances in the obtained snail extracts, on the other hand, on regenerative properties of the HaCaT human keratinocyte cell line is evaluated. In the frame of this invention, an object thereof is therefore the preparation of an extract from *Cornu aspersum* that brings a solution to the abovementioned problems of snail mortality and extract centrifugation notably. In this respect, the dermatoprotective properties of extracts of *Cornu aspersum* are investigated. When looking for a suitable substance therefor, such as for skin diseases or disorders, said proposed substance from the relevant group was tested.

### Summary of the invention

The invention is defined in the claims.

There is thus proposed according to the invention a method for snail extract acquisition comprising at least two steps, which is remarkable in that a first step for obtaining the extract consists of (i) a non-stressful snail extract acquisition, wherein a batch of snails is selected, which are brought to move by crawling on a set of glass surface means made of inert material consisting of glass plates, yielding a quantity of clear pedal mucus, which is removed from said surface means and placed into receptacles, and which is followed by a further step, wherein said further step consists of (ii) drying with a subsequent resuspension step of the produced snail extract. wherein the dried snail extract is further filtered through a microporous filter having a pore size of about 0,22µm.

In this respect, a method that is non stressful, in contrast with snail centrifugation, was thus tested in order to obtain clear pedal mucus from snails crawling on surfaces, made of inert material namely glass. Then drying and a subsequent resuspension of the snail extract is tested comparatively to extract centrifugation, on both snail extracts obtained either by centrifuged or crawling snails.

Snail mucus obtained either by centrifugation or by snail crawling on glass plates yet exhibit a significant positive effect in the regenerative properties of the cells in vitro. However, according to the results of the tests performed according to the invention, further treatment of the snail extract, that is dried versus centrifuged snail extract increased significantly the proliferation rate, the migration and wound healing potential, as well as the expression of protein-markers of cell survival, cell-cell and cell-substrate adhesion. This is presently attributed to the presence of growth factors that are more effective in terms of composition or concentration and/or antimicrobial substances in dried snail mucus, compared to the supernatant of centrifuged snail mucus.

According to this invention, said further step is thus remarkable in that it consists of drying with a subsequent re-suspension step of the produced snail extract. Dried snail extracts increase significantly the proliferation rate, the migration and wound healing potential of HaCaT cells, as well as the expression of protein-markers of cell survival, cell-cell and ceil-substrate adhesion in vitro, compared to said known centrifuged extracts.

In a particular embodiment of the method according to the invention, a snail type is selected from the gastropod family of *Cornu aspersum*, an extract is acquired from the snails, wherein a fraction is extracted therefrom, wherein a snail extract is derived from said fraction thereof. In a more particular embodiment of the method according to the invention, there is further carried out an evaluation of the effect of all snail extracts acquired, on regenerative properties of the HaCaT human keratinocyte cell line, wherein there is carried out an evaluation of the effect of the extracts on the HaCaT cell line by estimating the proliferation rate, the migration and wound healing potential, as well as the expression of protein-markers of cell survival (β-catenin), cell-cell adhesion (E-cadherin) and cell-substrate adhesion (integrin-β1), wherein the response of the HaCaT cell line treated with snail extracts under H₂O₂ induced oxidative stress is furthermore estimated, and wherein the potential protective or therapeutic role of bioactive substances in snail extracts is evaluated.

In a still more particular embodiment of the method according to the invention, said snails are arranged to crawl or walk under optimum conditions of moisture, temperature and duration, which are defined as a high humidity and room temperature of approximately 18° to 22°, particularly of about 20°C, on said surface means, being selected from glass plates, for about 2 hours, wherein further treatment of the snail extracts obtained by snail crawling (W-'walking') consists of drying and subsequent resuspension of the dried snail extract (dry), particularly wherein said receptacles consist of petri dishes of glass.

Then, the resuspended dry extract is filtered through a microporous filter having a pore size of about 0,22 µm; particularly wherein the Dry extract is obtained by placing the snail extracts into a set of inert substantially smooth plates, particularly glass petri dishes, into a chamber at approximately 40°C a certain duration corresponding thereto, notably of about 24h; more particularly wherein the dried snail extract is further resuspended in aqueous solution, notably with a pH of about 7,4, and filtered through a microporous filter having said pore size.

In a preferred embodiment of the method according to the invention said snail extracts are acquired essentially at any of the three sampling seasons, which are determined either as
(a) during a period of reproductive activity, essentially circumscribed between the months of September to November in the area of origin being the Mediterranean, resp.
(b) during a period of hibernation between the months of December to February and/or
(c) during a period of activity between the months of March to May;

particularly in that said snail extracts are acquired at the optimum sampling season for snail extracts being any of both sampling seasons mainly during said period of reproductive activity of snails (a), and also during said period of activity (c);
possibly in that said snail extracts are acquired when snail mucus is produced by snails during activity, either reproductive or not, as for defense and locomotion of gastropods that are exposed to the external environment.

Based on the annual life cycle of *Cornu aspersum* and in order to evaluate the optimum sampling season, snail extracts are acquired at said three sampling seasons: October as a period of reproductive activity, February as a hibernation period, and May as activity period. Noticeably, although this extract obtained from hibernating snails did not appear to influence cell proliferation or migration, it was effective after strong chemical oxidative stress as a therapeutic factor.

As skin ageing, skin exposure to various damaging environmental factors, or even skin burns could result in oxidative stress of human epithelial cells, this extract is further proposed according to the invention to be further used not only in cosmetology but also as a pharmaceutical product.

According to a limited embodiment of the invention, in a first instance, specific animal parts of *Cornu aspersum* are used, especially those less or not at all affected by the season. The result is that harvesting these offers the advantage of providing availability year-around thanks to appropriate storage conditions, and it also has been experimentally established and implemented in the meaning that the extract can be stored first and used later without losing its properties.

In a quite valuable embodiment of the method according to the invention, it is started from a snail extract of *Cornu aspersum* of natural origin, or from a cultured origin, particularly applied in a large scale such as in cooperation with snail farms. Further increase of crawling time would lead to an increase in the quantity of the snail extracts obtained, in case of commercial exploitation.

There is further achieved a snail extract obtained according to a method as proposed by the invention for use in the treatment of a disease relating to skin ageing, skin exposure to various damaging environmental factors, or skin burns.

The invention also relates to a snail extract obtained according to a method according to the invention notably as set out above for use in the treatment of a disease relating to skin ageing, skin exposure to various environmental factors, or skin burns.

There is further proposed according to the invention a cosmetic use of the snail extract obtained according to a method of the invention.

In addition, there is also proposed a pharmaceutical product comprising the snail extract obtained according to a method as proposed by the invention.

Finally, there is proposed a snail extract for use according to the invention, wherein the secretion of snails is used in the regeneration of burnt skin and in the management of open wounds. Additional features and particularities of the method according to the invention are defined in the additional sub-claims. Further details are incorporated in the following description of some preferred exemplary embodiments of the method according to the invention, which is illustrated based on the attached drawings.

### Brief description of the drawings

Fig. 1 shows a schematic representation of experimental design for the method according to the invention.
Fig. 2 shows a graphical representation of the proliferation rate of HaCaT cell line under the effect of snail extracts obtained from live adult snails during their reproductive period (GB).
Fig. 3 shows likewise an analogue representation thereof during hibernation (A).
Fig. 4 shows a graphical representation of the percentage of 'Wound' healing (mean ± standard deviation) potential of the human keratinocyte cell line HaCaT after treatment with snail extracts obtained from live adult snails during the reproductive and activity period.
Fig. 5 shows a graphical representation of the relative expression of E-cadherin, β-catenin and integrin-β1 in the human keratinocytes cell line HaCaT after the effect of extract from (A) centrifuged snails from the 1st sampling period further being treated by centrifugation (CGB CF) or drying (CGB dry), and (B) from crawling snails from the 1^{st} sampling period further being treated by centrifugation (WGB CF) or drying (WGB dry).
Likewise Fig. 6 shows a similar representation thereof, but after the effect of extract from centrifuged (CA dry) and crawling (WA dry) snails from the 2^{nd} sampling period further being treated by drying.
Again Fig. 7 shows an analogue representation thereof but after the effect of mucus from centrifuged (CE dry) and crawling (WE dry) snails from the 3^{rd} sampling period further being treated by drying.

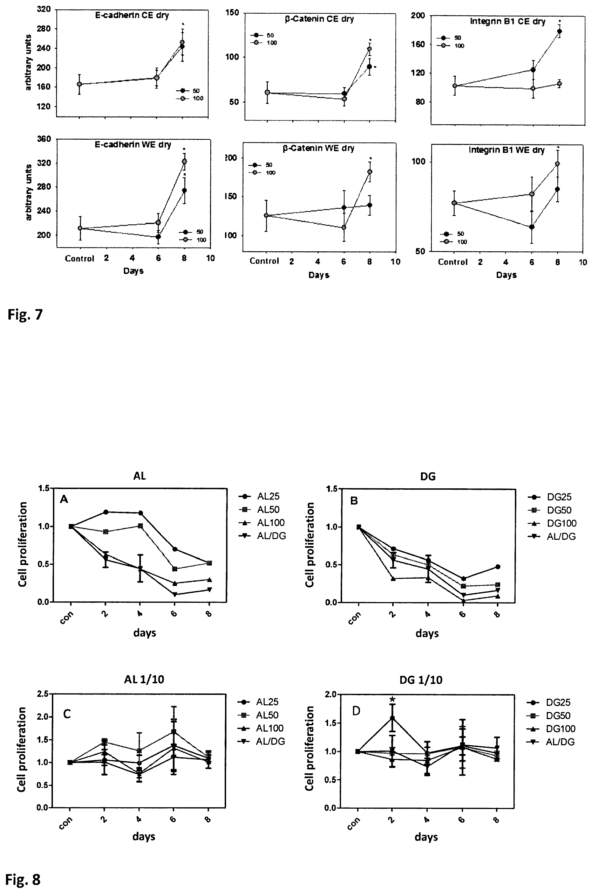

Fig. 8 similarly shows a graphical representation of the proliferation rate of HaCaT cell line, but under the effect of the albumen (AL) and digestive tract and gland (DG) extract obtained from snails during their reproductive period.

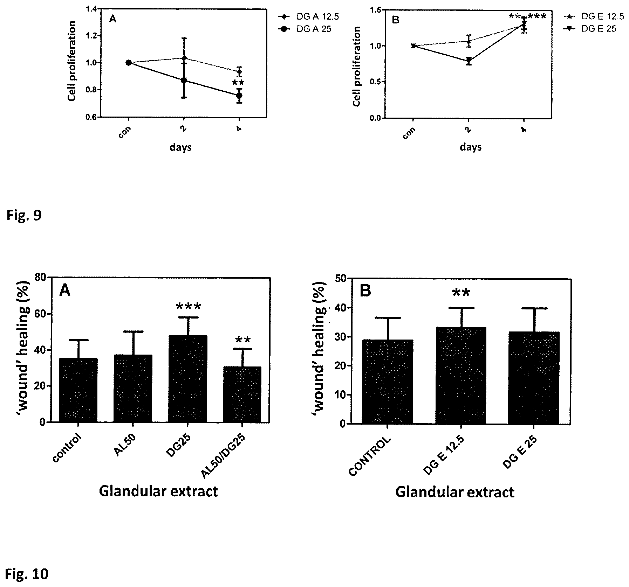

Fig. 9 shows an analogue representation thereof as in Fig. 8, but under the effect of the DG extract diluted by a factor 1/10 obtained from snails during (A) hibernation (DGA), (B) activity (DGE).
Fig. 10 shows a graphical representation of the percentage of 'wound healing' -mean ± standard deviation- potential of the human keratinocyte cell line HaCaT after treatment with snail extracts obtained from the albumen (AL) and digestive tract and gland (DG) of (A) the reproductive period, and (B) their period of activity.

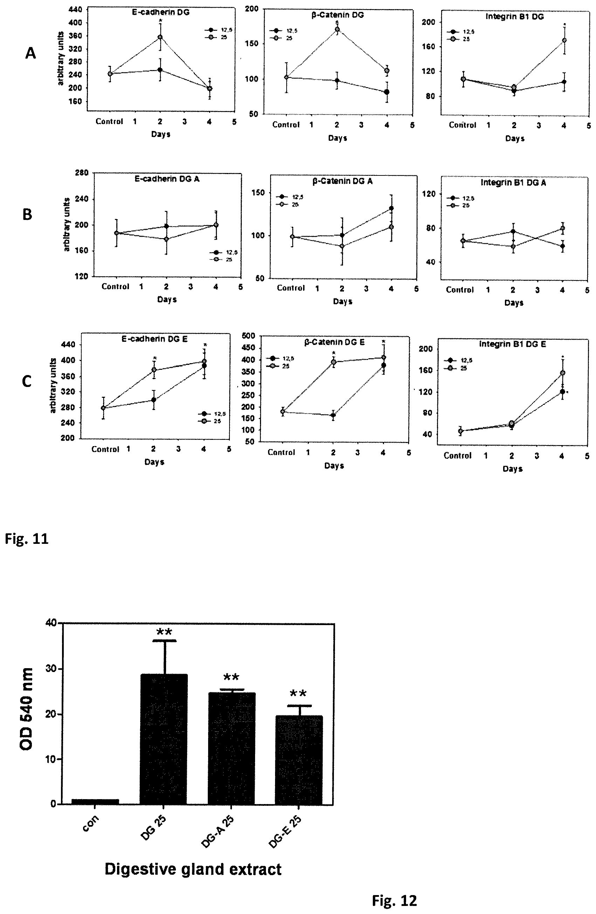

Fig.11 shows a relative expression of E-cadherin, β-catenin and integrin-β1 in the human keratinocytes cell line HaCaT after the effect of DG extract from A. the 1^{st}, 2^{nd} and 3^{rd} sampling periods.
Fig. 12 shows the therapeutic role -mean ± standard deviation- of DG extracts on the human keratinocyte cell line HaCaT after H₂O₂ induced oxidative stress, wherein treatments after oxidative stress were performed with 1/10 diluted DG extracts derived from snails during said three sampling periods.

### Description

With this invention, an improved method is developed for a snail extract acquisition process, respective the most commonly used method until now. In this respect, a non stressful method was tested in order to obtain clear pedal mucus from snails crawling on glass surfaces in comparison to snail centrifugation which may result in snail mortality. A drying and subsequent re-suspension of the snail extract, comparatively to extract centrifugation, was tested on both snail extracts obtained by crawling snails, but also centrifuged ones. The effect of specific glandular extracts namely, albumen gland of the reproductive system and proximal part of the digestive tract of the snails on regenerative properties of the HaCaT human keratinocyte cell line was further evaluated. The effect of the specific extracts on the HaCaT cell line was evaluated by estimating the proliferation rate, the migration and wound healing potential, as well as the expression of protein-markers of cell survival (β-catenin), cell-cell adhesion (E-cadherin) and cell-substrate adhesion (integrin-β1). Furthermore, in order to evaluate the potential protective or therapeutic role of bioactive substances in snail extracts, the response of the HaCaT cell line treated with snail extracts under H₂O₂ induced oxidative stress was estimated.

An optimal method of snail extracts acquisition from *Cornu aspersum* and investigation of their regenerative properties is tested.

Regenerative properties of the human keratinocyte cell line HaCaT appear to be positively influenced by factors in (**A**) dried snail extract and (**B**) extracts of the proximal parts of the digestive tract from *Cornu aspersum* snails.

Said known method of snail extract acquisition, consists of two steps, including a first centrifugation of the snails in order to obtain the extract and a further centrifugation of the snail extract. The supernatant of the second centrifugation is used in cosmetology. The effect of the bioactive substances in the obtained snail extracts on regenerative properties of the HaCaT human keratinocyte cell line is evaluated.

The results obtained with this invention reveal that both processes of snail extract acquisition being snail centrifugation vs. snail crawling on glass plates, yield extracts that exhibit a positive effect in the regenerative properties of the cells *in vitro.* It's the further treatment of both snail extracts that differentiate their regenerative effect. Dried snail extracts according to the invention increase significantly the proliferation rate, the wound healing potential of the cells, as well as the expression of all protein-markers tested, compared to the known centrifuged extract.

As to the snail extract acquisition, the secretion of the mollusk *Cornu aspersum* is a natural product that bears regenerative properties when applied topically on skin. An initial step in this experimental procedure was to apply the extraction method used to date as presented in said US - 5538740 on adult snails.

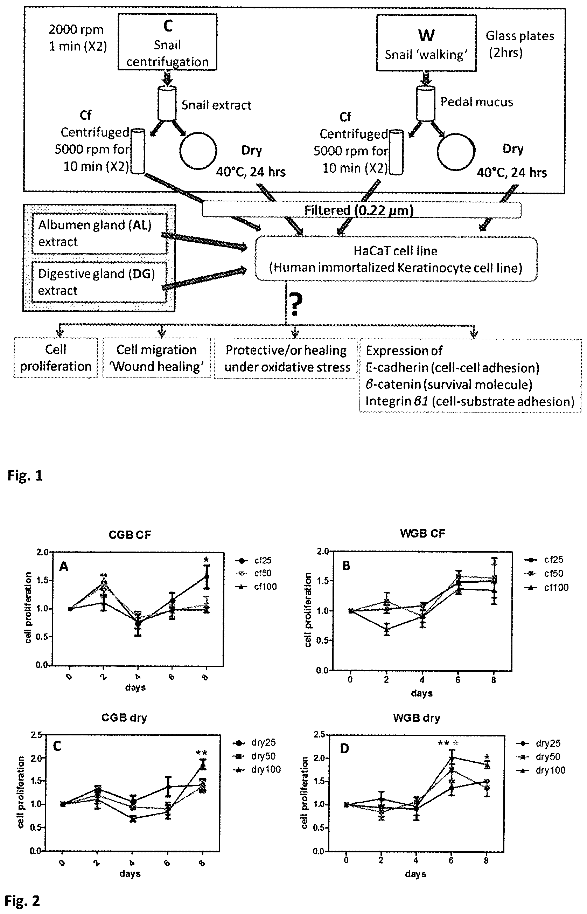

Fig. 1 shows a schematic representation of experimental design, wherein C stands for centrifuged snails, **W** for 'walking' snails, **cf** for centrifuged snail extracts, **dry** for dried snail extract. The experimental procedure was repeated on three sampling seasons: **GB** for October, period of reproductive activity, **A** for February-hibernation, and resp. **E** for May, activity period.

A non stressful method of snail extract acquisition was tested. According to this method, snails were allowed to crawl or 'walk' on a set of inert plates under optimum conditions, being defined as e.g. high humidity and 20°C on the e.g. glass plates for about 2 hours. Clear pedal mucus was carefully removed from said glass plates and placed into glass petri dishes. Further treatment of the snail extracts obtained either by said snail centrifugation **C**, or snail crawling **W-**'walking' consists of (a) centrifugation **cf** and (b) drying and subsequent resuspension of said dried snail extract **dry** shown in Fig. 1. Specifically, **cf** extract was obtained after two successive centrifugations at 5000 rpm for 10 minutes. The final supernatant was then separated and filtered through a microporous filter having a pore size of 0,22 *µ*m. **Dry** extract was obtained by placing the snail extracts into glass petri dishes into a chamber at 40°C for 24h. The dried snail extract was further resuspended in aqueous solution, typically with pH 7,4 and filtered through a microporous filter having a pore size of 0,22 *µ*m.

As comparative example, two specific glandular extracts were tested for their effect on the HaCaT human keratinocyte cell line. For this purpose, tissue samples were isolated from: the albumen gland **AL** with the embedded FPSC from the reproductive system, and the proximal part of the digestive tract and gland **DG** in a number of snails wherein n=30. Tissue samples were homogenized and centrifuged at 5000 rpms for 10 min. The supernatant of these samples was used as bioactive substances in HaCaT cell cultures visible in Fig. 1. All samples of snail extracts were stored at -25° C until use.

Based on the annual life cycle of *Cornu aspersum* and in order to evaluate the optimum sampling season, snail extracts were acquired at three sampling seasons: (a) October **GB** -period of reproductive activity, (b) February **A**-hibernation, and (c) May **E**-activity period.

The toxicity of each sample was assayed by the trypan blue exclusion method and the highest effect/toxicity ratio was defined in order to employ the specific concentration for the in vitro assays on HaCaT cell line.

With regard to the materials and methods involved, the evaluation of the effect of specific snail extracts on the regenerative properties of HaCaT cells, and the determination of the optimum extract in terms of tissue specific, time and dosage dependent treatment, was tested in four levels: **(a)** the induction of cell proliferation ; **(b)** the stimulation of cell migration ; **(c)** the protection and/or healing role under oxidative stress and **(d)** the increase of the expression of ECD (cell-cell adhesion molecule), β-catenin (survival molecule), β1-integrin (cell-substrate adhesion molecule). Each level is set out in detail below:
as to cell proliferation **(a)**, HaCaT cells (CLS order No. 300493) are a human keratinocyte cell line used in scientific research. They are utilized for their high capacity to differentiate and proliferate in vitro. Cells were grown in T-75 flasks using Dulbecco's modified Eagle's medium containing 10% (v/v) FBS (fetal bovine serum) and 5% penicillin/streptomycin (1/1). Cells were incubated at 37°C in an atmosphere containing 5% CO₂. HaCaT cells cultured on 6-well or 12-well Petri dishes were treated with different concentrations of snail extracts at 0, 25, 50 and 100 µg/ml during several time points: 2, 4, 6 and 8 days. In order to estimate cell proliferation and cell viability, culture samples were transferred to a Neubauer chamber counter and were examined under a light microscope using the trypan blue exclusion method.

As to cell migration **(b)** brings wound healing, HaCaT cells cultured on 6-well Petri dishes were treated with said concentrations of snail extracts at 0, 25, 50 and 100 µg/ml during different several time points of 2, 4, 6 and 8 days. *In vitro* wound-healing assays were initially performed as previously described (*Brieva et al 2008*). Briefly, HaCaT were seeded in T6-well culture dishes at a density of 3·10⁵ cells per well. Confluent cell monolayers were then gently scratched with a pipette tip across the entire diameter of the dish. Next, cells were rinsed with medium to remove all cellular debris, and treatment with snail extracts was applied to three wells, whereas the rest of the wells served as control. Cultures were observed immediately after wounding and 24 h and 48 h later, and phase-contrast pictures were taken of the wounded area using a high-resolution microscopic camera connected to the microscope. Quantification of migrated cells was measured in the healed area.

The effect of snail extracts on wound healing capability and cell migration potential of HaCaT cells could not be comparatively studied, due to the fact that cell migration was rapid within 24 h in all treatments, with or without snail extracts. Therefore, it was tried to increase the wound and the time of healing. Specifically, cell monolayers in p60 Petri dishes were gently scratched using a cell scraper with diameter 1,5 cm across the entire diameter of the dish. According to the results previously obtained from cell proliferation, the effect of specific concentrations was tested at a time period of 8 days. In order to avoid the accumulation of toxic substances in the cell medium during this prolonged time period, every two days the medium was replaced with new, whereas snail extracts were also renewed. Quantification of migrated cells was measured in the healed area by high-resolution camera connected to the stereoscope (Zeiss 2000-C) using the AxioVision Rel. 4.7 software.

As to protective/healing role under oxidative stress **(c)**, according to the oxidative stress model (Liu et al 2012), treatment with different concentrations of H₂O₂ ranging from 0,1 to 1 mM for different time periods of 0, 0,5, 1, 2, 4 hours on HaCaT cells was tested in order to establish the IC50 for H₂O₂, that is treatment (concentration and time of effect) which results in 50% mortality in HaCaT cells. Cell viability was documented by the MTT assay (Merlin et al 1992). Cells were treated with the abovementioned concentrations of H₂O₂ and 0,5 ng/ml of MTT solution was added. Cells were then incubated at 37°C for 2-3 h and the resulting formazan crystals were dissolved by the addition of Dimethyl sulfoxide (DMSO). Absorbance was measured at 540 nm. IC50 was established with 0,75 mM H₂O₂ for 2 hours. In order to determine whether snail extracts perform a protective and/or a healing effect on cells that undergo oxidative stress, two different approaches were applied: at first the protective role of snail extract including, treatment with the optimum snail extract respective concentration and time of effect; Induction of oxidative stress for IC50 (0,75 mM H₂O₂ for 2 hours) and Documentation of cell viability by the MTT assay, resp.

The other approach about the healing role of snail extract includes induction of oxidative stress for IC50 (0,75 mM H₂O₂ for 2 hours); removal of cell medium, cell washing, and replacement with new cell medium; treatment with the optimum snail extract (concentration and time of effect); and documentation of cell viability by the MTT assay.

As to expression of E-cadherin (cell-cell adhesion), *β*-catenin (survival molecule) and integrin-*β1* (cell-substrate adhesion) **(d)**: during the first sampling season in October the expression of the particular protein-indicators in the cells after the effect of all extract samples was studied. All the extracts were tested irrespective of the way they were obtained either **C**, **W**, **AL** and **DG** and further treated **cf** and **dry** in all concentrations at 25, 50 and 100 µgr/ml and time periods 2, 4, 6 and 8 days used. Judging from the cell proliferation results of the first sampling season, the extracts obtained from snails during the two following sampling seasons were tested only at the concentrations and time periods that gave the optimum effect. Specifically, the effect of dried extract dry at 50 and 100 µgr/ml after 6 and 8 days and the effect of digestive tract and gland **DG** at 12,5 and 25 µgr/ml after 2 and 4 days, were tested.

Cells were homogenized in cold lysis buffer (20 mM β-giycerophosphate, 50 mM NaF, 2mM EDTA, 20 mM Hepes ((4-(2-hydroxyethyl)-1-piperazineethanesulfonic acid), 0,2 mM Na₃VO₄, 10 mM benzamidine, pH 7, containing 200 µM leupeptin, 10 µM trans-epoxy succinyl-L-leucylamido-(4-guanidino) butane, 5 mM DTT (dithiothreitol), 300 µM phenyl methyl sulfonyl fluoride (PMSF) and 1% v/v Triton X-100), and extracted on ice for 30 min. Samples were centrifuged (10.000 g, 10 min, 4°C) and the supernatants were boiled with 0,33 volumes of SDS/PAGE sample buffer [330 mM Tris-HCl, 13% v/v glycerol, 133 mM DTT, 10% w/v SDS (Sodium Dodecyl Sulfate), 0,2% w/v bromophenol blue]. Protein concentration was determined using the BioRad protein assay.

Equivalent amounts of protein (100 µg) were separated on 10% (w/v) acrylamide, 0,275% (w/v) bisacrylamide slab gels and transferred electrophoretically onto nitrocellulose membranes (0,45 µm, Schleicher and Schuell, Keene N. H. 03431, USA). Non-specific binding sites on the membranes were blocked with 5% (w/v) non-fat milk in TBST (Tris Buffered Saline-Twin 20) (20 mM Tris-HCI, pH 7,5, 137 mM NaCl, 0,1% (v/v) Tween 20) for 30 min at room temperature. Subsequently, the membranes were incubated overnight with the appropriate primary antibodies. Antibodies used were as follows: Anti-E-cadherin, clone DE CMA-1, Anti-β-Catenin and Anti-Integrin B1 (Merck). After washing in TBST (3x5 min), the blots were incubated with horseradish peroxidase-linked secondary antibodies, polyclonal goat anti-mouse immunoglobulins and polyclonal goat anti-rabbit immunoglobulins (DAKO, High Wycombe, Buckinghamshire, UK), washed again in TBST (3x5min), and the bands were detected by enhanced chemiluminescence (Cell Signaling, Beverly, MA, USA) with exposure to Fuji Medical X-ray films. Films were quantified by laser-scanning densitometry (GelPro Analyzer Software, Media Cybernetics) and blots were plotted using SigmaPlot 10.0.

The results of the tests set out above are as follows: firstly, improving the snail extract processing: the dried snail extract increased regenerative properties of HaCaT cells versus the centrifuged one.

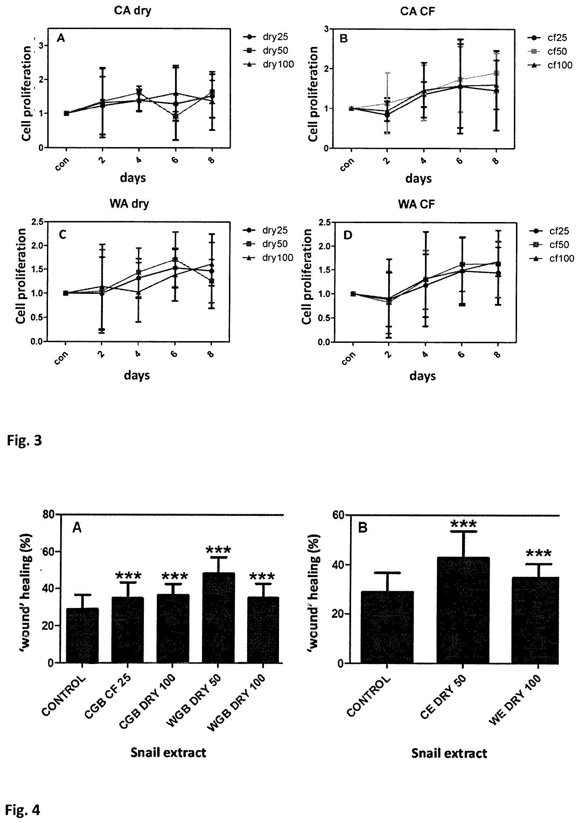

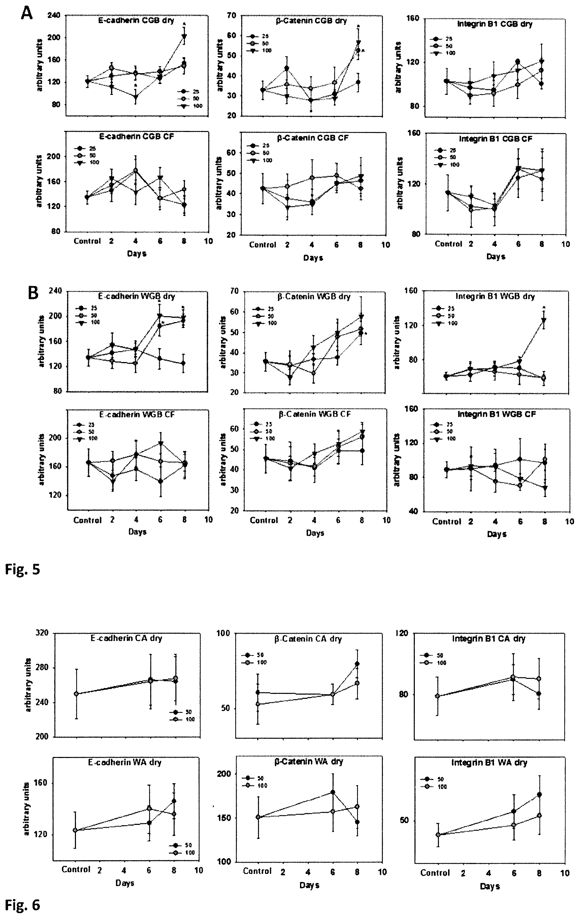

However, according to the results, snail extracts obtained either by centrifugation (**C**) or by snail crawling on glass plates (**W**), both exhibited a significant positive effect at the regenerative properties of the cells *in vitro,* it was the processing of the snail extract that is dried instead of the centrifuged snail extract, that further increased significantly: the proliferation rate a shown in Fig. 2, the migration and wound healing potential as shown in Fig. 4, as well as the expression of protein-markers of cell survival, cell-cell and cell-substrate adhesion as shown in Fig. 5 of HaCaT cells.

Indeed, Fig. 2 shows the proliferation rate of HaCaT cell line under the effect of snail extracts obtained from live adult snails during their reproductive period (**GB**). **CGB** and **WGB** refer to centrifuged and 'walking' snails, respectively; **CF** to centrifuged snail extract, **dry** to dried snail extract. Numbers represent treatment concentrations in µgr/ml.

The proliferation rate of HaCaT cells appeared to be positively influenced under the effect of snail extracts obtained from live adult snails during the first sampling season, reproductive period-**GB**. In general, the proliferation rate increased two days after treatment, following a decrease close to the proliferation rate of the control group, while any statistically significant effect of treatment was observed after 6 and 8 days (CGB CF 25, CGB dry 100, WGB dry 50 & 100). Noticeably, the same sample of snail extract, either CGB visible in Fig. 2-A,C or WGB in Fig. 2-B,D, induced significantly cell proliferation when dried, while this positive influence was not observed when the snail extract was centrifuged.

On the contrary, as shown in Fig. 3, all snail extracts obtained during the second sampling season in February-**A** from hibernating snails, demonstrated no significant positive effect on cell proliferation rate. Indeed Fig. 3 shows the proliferation rate of HaCaT cell line under the effect of snail extracts obtained from live adult snails during hibernation (**A**). **CA** and **WA** refer to centrifuged and 'walking' snails, respectively. **CF** stands for centrifuged snail extract, resp. **dry** for dried snail extract. Numbers represent treatment concentrations (µgr/ml).

In order to corroborate these results, snail extracts of the second sampling season were used in treatments testing levels of protein expression (E-cadherin, β-catenin, integrin-β1), while they were not used as potential bioactive substances in estimating 'wound' healing, or their healing/protective role in induced oxidative stress experiments in HaCaT cells.

The results of cell proliferation obtained from extracts from active snails of the third sampling season May-**E** corroborated the results obtained so far. Dried snail extracts again increased significantly cell proliferation. Specifically, snail extract from centrifuged snails (**CEdry**) increased slightly cell proliferation rate (10%) at 50 µg/ml concentration, while it had no significant effect at the highest concentration. Snail extract from crawling snails (**WEdry**), also had a high effect on proliferation rate (50%) at the concentration of 100 µgr/ml. All significant effects were observed 8 days after treatment.

Fig. 4 shows the percentage of Wound healing (mean± standard deviation) potential of the human keratinocyte cell line HaCaT after treatment with snail extracts obtained from live adult snails during **A.** the reproductive period-October -**CGB** and **WGB** refer to centrifuged and walking snails, respectively- and **B.** during their activity period-May -**CE** and **WE** refer to centrifuged and 'walking' snails, respectively- **CF** stands for centrifuged snail extract, and **dry** for dried snail extract resp. Numbers represent treatment concentrations µgr/ml.

Cell migration of HaCaT cell line was significantly increased after treatment with snail extracts that were mainly dried. Specifically, wound healing potential of this cell line was significantly higher ranging from 34,7% to 48,3%, compared to the control (28,8% wound healing) when treated mainly with dried snail extracts obtained from snails during their reproductive period in October-**GB** as shown in Fig. 4-A. Similar results were obtained from dried extracts from active snails of the third sampling season in May-**E** shown in Fig.4-B with a higher 'wound' healing potential of CEdry50 (42,8%) and WEdry100 (34,5%) compared to the control (28,8%).

Fig. 5 shows the relative expression of E-cadherin, β-catenin and integrin-β1 in the human keratinocytes cell line HaCaT after the effect of extract from A. centrifuged snails from said 1^{st} sampling period further being treated by centrifugation (CGB CF) or drying (CGB dry), and B. from crawling snails from said 1^{st} sampling period further being treated by centrifugation (WGB CF) or drying (WGB dry). Numbers represent treatment concentrations in µgr/ml.

The results regarding the expression of protein-markers of cell survival, cell-cell and cell-substrate adhesion of HaCaT cells were in line with cell proliferation and wound healing results. Extracts from centrifuged snails being further treated with centrifugation (CGB cf) from said 1^{st} sampling period, had no effect on the expression of E-cadherin at all concentrations tested as shown in Fig. 5-A. On the other hand, the effect of the same extract further treated by drying and resuspension (CGB dry) resulted in a 2-fold increase in the expression levels of E-cadherin at the concentration of 100 µgr/ml on the 8^{th} day of the effect compared to the control. The same results were obtained regarding β-catenin. On the other hand, the expression of integrin-β1 remained unchanged under the effect of all bioactive substances.

Extracts from crawling snails being further treated by centrifugation (WGB cf), had no effect in the expression levels of E-cadherin at all concentrations as shown in Fig. 5-B. On the other hand, the effect of the same extract being further treated by drying and resuspension (WGB dry), resulted in a 1,7-fold increase of E-cadherin expression levels compared to the control, at the concentrations of 50 and 100 µgr/ml on the 6^{th} and 8^{th} day of the effect. WGB dry effect resulted also to a statistically significant increase (1,4-fold compared to the control) in the expression levels of β-catenin at the concentration of 25 µgr/ml on the 8^{th} day of the effect. Integrin-β1 levels increased 2-fold compared to the control, only under the effect of WGB dry 100 on the 8^{th} day of effect.

Fig. 6 shows the relative expression of E-cadherin, β-catenin and integrin-β1 in the human keratinocytes cell line HaCaT after the effect of extract from centrifuged (CA dry) and crawling (WA dry) snails from the 2^{nd} sampling period in February-A further being treated by drying. Numbers represent treatment concentrations in µgr/ml.

Concerning the effect of extracts obtained from snails of the 2^{nd} sampling period in February A, only the optimum time and dosage dependent treatments based on the results from the 1^{st} sampling in October-GB were applied: concentrations of 50 and 100 µgr/ml of extract further treated by drying after 6 and 8 days of effect. No change was observed in the expression levels of E-cadherin, β-catenin and integrin-β1 at all treatments tested. Specifically, under the effect of both used concentrations of 50 and 100 µgr/ml, no particular changes were observed in the expression of the 3 examined proteins on the 6^{th} and 8^{th} day of the effect.

Fig. 7 shows the relative expression of E-cadherin, β-catenin and integrin-β1 in the human keratinocytes cell line HaCaT after the effect of mucus from centrifuged (CE dry) and crawling (WE dry) snails from said 3^{rd} sampling period in May further being treated by drying. Numbers represent treatment concentrations in µgr/ml.

Concerning the effect of extracts obtained from snails of the 3^{rd} sampling period in May-E when the snails were active, increased expression levels of the examined proteins mostly on the 8^{th} day of the effect were observed. Specifically, the CE dry effect caused an increase in the expression levels of E-cadherin and β-catenin of 1,7-fold compared to the control in both examined concentrations of 50 and 100 µgr/ml. Integrin-β1 levels increased 1,8-fold compared to the control at the concentration of 50 µgr/ml on the 8^{th} day of the effect. WE dry effect resulted in increased β-catenin and integrin-β1 levels of 1,5 and 1,7-fold respectively compared to the control at the concentration of 100 µgr/ml on the 8^{th} day of the effect. The same concentration of the extract WE dry, resulted in a 1,5-fold increase in the expression levels of E-cadherin on the 8^{th} day of the effect. The concentration of 50 µgr/ml caused a 1,3-fold increase of the particular protein compared to the control levels, also on the 8^{th} day of the effect.

The extract from the proximal part of the digestive tract and gland increases the regenerative properties of HaCaT cells and plays a therapeutic role under strong chemical oxidative stress.

Fig. 8 shows the proliferation rate of HaCaT cell line under the effect of the albumen AL and digestive tract and gland (DG) extract obtained from snails during their reproductive period. Numbers represent treatment concentrations in µgr/ml.

Regarding proliferation rate, an initial treatment with specific snail extracts from the albumen AL and the proximal part of the digestive tract and gland (**DG**) demonstrated a negative effect on cell proliferation rate as shown in Fig. 8-A,B. Due to the fact that this experimental procedure was tested for the first time, and given that the albumen gland provides nutrients to the fertilized ova, while the digestive gland has been proposed to play a fundamental role in shell regeneration, lower concentrations of these snail extracts were tested. Thus, these specific extracts were diluted by a factor 1/10 and were further tested as shown in Fig. 8-C,D. The **AL** extract showed a positive effect 2 and 6 days after treatment, although it was not statistically significant as shown in Fig. 8-C. On the other hand, the **DG** extract resulted in a statistically significant increase in cell proliferation rate two days after treatment as shown in Fig. 8-D. Moreover, the combined effect (1/1) of these two glandular extracts **AL/DG** did not appear to influence positively cell proliferation rate.

Fig. 9 shows the proliferation rate of HaCaT cell line under the effect of the DG extract diluted by a factor 1/10 obtained from snails during A. hibernation DGA, B. activity DGE, Numbers represent treatment concentrations in µgr/ml.

Following the latter result, only diluted (1/10) **DG** extracts were tested from snails during the two following sampling periods DGA-February-hibernation and DGE-May-activity and for all other tests thereafter performed (wound healing, protein expression, oxidative stress). Additionally, a more diluted treatment (12,5 µgr/ml) was tested. The DGA extract influenced negatively the proliferation rate of HaCaT cellsas shown in Fig. 9-A. On the contrary, the DGE extract demonstrated a positive effect on cell proliferation rate 4 days after treatment in both concentrations of 12,5 and 25 µgr/ml of the initial diluted extract used as shown in Fig. 9-B.

Fig. 10 shows the percentage of wound healing (meant standard deviation) potential of the human keratinocyte cell line HaCaT after treatment with snail extracts obtained from the albumen (AL) and digestive tract and gland DG of **A.** the reproductive period in October, and **B.** their period of activity in May. Numbers represent treatment concentrations in µgr/ml.

Considering the wound healing capability of HaCaT cells, a positive effect was evident on the cells after treatment with the **DG** extract, while no positive effect was revealed after treatment with the **AL** extract both obtained from snails during the reproductive season as shown in Fig. 10-A. Moreover, the **AL** extract when combined to the **DG** extract (AL50/DG25) inhibited said wound healing effect of the latter extract (**DG25**) on HaCaT cells as shown in Fig. 10-A. Therefore, the **AL** extract was not used as a bioactive substance during the two following sampling seasons of February and May. In line with the previous results a positive effect was also demonstrated by the DG extract when obtained during the third sampling season (DGE) at a smaller concentration 12,5 µgr/ml as shown in Fig. 10-B.

Fig. 11 shows the relative expression of E-cadherin, β-catenin and integrin-β1 in the human keratinocytes cell line HaCaT after the effect of **DG** extract from **A.** the 1^{st} sampling period in October-**DGB**. the 2^{nd} one in February-**DGA**, and **C.** the 3^{rd} one in May-**DGE**. Numbers represent treatment concentrations in µgr/ml.

Considering protein expression levels, **DG** extract from snails of said first sampling period of reproductive activity in October applied on HaCaT cells increased the expression levels of E-cadherin and β-catenin (1,5 and 1,8-fold compared to the control) two days after treatment at the concentration of 25 µgr/ml. The expression level of integrin-β1 increased 1,4-fold compared to the control four days after treatment at the same concentration. The concentration of 12,5 µgr/ml had no effect on the expression levels of the examined proteins as shown in Fig. 11-A.

The effect of the **DG** extract from snails of said second sampling period of hibernation-DGA in February, had no effect on the expression levels of E-cadherin, β-catenin and integrin-β1. Specifically, both concentrations of 12,5 and 25 µgr/ml had no effect on the expression levels of the examined proteins on the 2^{nd} and 4^{th} day of the effect as shown in Fig. 11-B.

Contrarily to the above obtained results, the effect of **DG** extract from active snails of said third sampling period of activity period-DGE in May resulted in a statistically significant increase of the expression levels of the examined proteins mostly four days after treatment at both concentrations. Moreover, two days after treatment, the expression levels of **E**-cadherin and β-catenin were statistically significantly increased. Specifically, E-cadherin expression levels were increased 1,4-fold compared to the control levels on the 2^{nd} day of effect at the concentration of 25 µgr/ml and on the 2^{nd} and 4^{th} day of effect at both concentrations used of 12,5 and 25 µgr/ml. β-catenin exhibited a similar pattern of expression under the same effects and the same time course, with its levels being 2-fold increased compared to the control. On the contrary, integrin-β1 levels increased by 3-fold compared to control levels only on the 4^{th} day of the effect at both concentrations as shown in Fig. 11-C.

Fig. 12 shows the therapeutic role (mean± standard deviation) of **DG** extracts on the human keratinocyte cell line HaCaT after H₂O₂ induced oxidative stress 0,75 mM H₂O₂ for 2h. Treatments after oxidative stress were performed with 1/10 diluted **DG** extracts derived from snails during the three sampling periods in DG25-October, DGA25-February, DGE25-May.

For the first time, the extract of the proximal part of the digestive tract and gland has been recognized as a potential therapeutic factor for HaCaT cells under H₂O₂ induced oxidative stress. Specifically, extracts obtained from snails during all sampling seasons DG in October-reproductive activity, DGA in February-hibernation, DGE in May-activation increased significantly cell viability after H₂O₂ induced oxidative stress at the rate of 0,75 mM for 2h. The capability of HaCaT cells to overcome this strong oxidative stress, which led to IC50, i.e. with 50% cell mortality, was apparent after the up to 30-fold increase of cell viability and proliferation two days after treatment with the DG extracts as shown in Fig. 12.

The experimental results above yield the following considerations: regenerative properties of the human keratinocyte cell line HaCaT appear to be positively influenced by factors mainly in dried snail extracts, on the one hand, and in snail extracts of the proximal part of the digestive tract and gland, on the other hand.

Until now, the most commonly used method in snail extract acquisition consists of two steps, a first centrifugation of the snails in order to obtain the extract and a further centrifugation of the snail extract. The supernatant of the second centrifugation is used in cosmetology. In this development, an attempt was made to develop alternative methodology for both steps of the already used process of snail extract acquisition and evaluate the effect of the bioactive substances in the obtained snail extracts on regenerative properties of the HaCaT human keratinocyte cell line. Specifically **(i)** a non-stressful method, was tested in order to obtain clear pedal mucus from snails crawling on glass surfaces, and **(ii)** drying and subsequent resuspension of the snail extract, was tested on both snail extracts obtained either by centrifuged or crawling snails. Dried snail extracts increased significantly the proliferation rate, the migration and wound healing potential of HaCaT cells, as well as the expression of protein-markers of cell survival, cell-cell and cell-substrate adhesion *in vitro,* compared to centrifuged extracts. According to these results, although snail mucus obtained either by centrifugation or by snail crawling on glass plates, exhibited a significant positive effect in the regenerative properties of the cells *in vitro,* further treatment of the snail extract, that is dried versus centrifuged snail extract increased significantly the proliferation rate, the migration and wound healing potential, as well as the expression of protein-markers of cell survival, cell-cell and cell-substrate adhesion. This is presently attributed to the presence of more effective growth factors in terms of composition or concentration and/or antimicrobial substances in dried snail mucus, compared to the supernatant of centrifuged snail mucus.

A second part of the disclosure concerns the evaluation of the effect of specific glandular extracts albumen gland of the reproductive system, proximal part of the digestive tract of the snails on regenerative properties of the HaCaT human keratinocyte cell line. For the first time, the bioactive potential of the glandular extract from the proximal part of the digestive tract of *Cornu aspersum* was identified at all levels of this process. This extract appeared to induce significantly the proliferation rate, the migration and wound healing potential, as well as the expression of all protein-markers tested. Among all extracts tested, only this specific glandular extract obtained by snails of all sampling seasons, i.e. October-reproductive activity, February-hibernation, May-activation, demonstrated a significant therapeutic role on cells under H₂O₂ induced oxidative stress *in vitro.* Noticeably, although this extract obtained from hibernating snails did not appear to influence cell proliferation or migration, it was effective after strong chemical oxidative stress as a therapeutic factor. This initial paradox observation is presently attributed to the physiological role of this gland during hibernation as it could maintain an antioxidant role in snails with low metabolic rates. In addition, the therapeutic role of this extract obtained from active snails is presently explained by the presence of regenerative factors in its glandular secretions as regeneration of epithelial cells is mandatory. The bioactive potential and therapeutic role of this glandular extract was apparent yet in extremely low concentrations. As skin ageing, skin exposure to various damaging environmental factors, or even skin burns could result in oxidative stress of human epithelial cells, this glandular extract can be further used not only in cosmetology but also as a pharmaceutical product.

Based on the annual life cycle of *Cornu aspersum* and in order to evaluate the optimum sampling season, snail extracts were acquired at three sampling seasons: October-period of reproductive activity, February-hibernation, and May- activity period. According to the results obtained, the optimum sampling season for snail extracts was mainly during reproductive activity of snails (October), and also on May when snails were active. On the other hand, snail extracts obtained from snails during hibernation did not exhibit regenerative properties, which could be explained by the decrease in metabolic rate of snails during hibernation *(Guppy and Withers, 1999).* Regenerative properties of snail extracts obtained during reproductive activity are presently attributed to the presence of growth factors and antimicrobial substances in snail mucus composition. It is known that *Cornu aspersum* performs multiple mating with different mating partners during a reproductive season. During prolonged mating events ranging from 2-8 hours, *(Koemtzopoutos & Staikou 2007, Garefalaki 2010),* snails are exposed to infections as they do not move and expose parts of their reproductive system to the environment. Moreover, dart shooting during copulation could potentially drive the evolution of a wound healing mechanism in this species. This mechanism could act through the release of regenerative factors during the reproductive period. In general, snail mucus produced by snails during activity reproductive or not is crucial in defense and locomotion of gastropods that are exposed to the external environment *(Denny 1980, Davies & Hawkins 1998).*

### References

Ansart A., Vernon P., Daguzan J. 2002. Elements of cold hardiness in α littoral population of the land snail Helix aspersa (Gastropoda: Pulmonata). Journal of Comparative Physiology B, 172, 619-625.

Ashcroft GS, Horan MA, Ferguson MW (1995)The effects of ageing on cutaneous wound healing in mammals. J. Anat. 187(Pt 1) 1-26.

Badiu DL, Balu AM, Barbes L, Luque R, Nita R, Radu M, Tanase E, Rosoiu N (2008). Physico-chemical characterisation of lipids from Mytilus galloprovincialis (L.) and Rapana venosa and their healing properties on skin burns. Lipids 43, 829-841.

Barnhart M.C. & McMahon BR (1987) Discontinuous CO2 release and metabolic depression in dormant land snails. Journal of Experimental Biology, 128: 123-138.

Baron-Barthelemy M. Mémoire sur les Preparations à Base d'Hélicine Admises à l'Exposition Universelle de 1855. Sirop et Bonbons Héliciés. De Bailly, Divry et Cie, Paris, 1855.

Bonnemain B. Helix and drugs: snails for health care from Antiquity to these days. Rev Hist Pharm (Paris) 2003;51(338):211-8. Brieva A, Philips N, Tejedor R, Guerrero A, Pivel J.P., Alonso-Lebrero J.L., Gonzalez S. Molecular Basis for the Regenerative Properties of a Secretion of the Mollusk Cryptomphalus aspersa. Skin Pharmacol Physiol 2008;21:15-22.

Brooks SA, Leatherm AJ. Prediction of lymph node involvement in breast cancer by detection of altered glycosylation in the primary tumour. Lancet 1991;38:71-4.

Calow P (1978) Why some metazoan mucus secretions are more susceptible to microbial attack than others (1978) The Americal Naturalist, 149-152.

Cottrell JM, Henderson IF, Pickett JA, Wright DJ (1993) Evidence for glycosaminoglycans as a major component of trail mucus from the terrestrial slug Arion ater L. Comparative Biochemistry and Physiology, 104B, 455-468.

Cranga F, Cranga Y. L'Escargot: Zoologie, Symbolique, Imaginaire, Médecine et Gastronomie. du Bien Public, Dijon, 1991. Davies MS, Hawkins SJ (1998) Mucus from marine molluscs. Advances in marine biology, 34, 2-71. Academic Press.

Denny MW (1980) Locomotion: the cost of gastropod crawling. Science 208: 1288-1290.

Denny MW (1989) Invertebrate mucous secretions: functional alternatives to vertebrate paradigms. In: Symposium XLIII of the Society for Experimental Biology; Mucus and related Topics. (E. Chantler & NA Ratcliffe eds), 337-366.

Deyrup-Olsen I, Louie H, Martin AW, Luchtel DL (1992). Triggering by ATP of product release by mucous granules of the land slug Ariolimax columbianus. American Journal of Physiology,232,760-765.

Dittbrenner N, Lazzara R, Koehler HR, Mazzia C, Capowiez Y, Triebskorn R (2009) Heat tolerance in Mediterranean land snails: Histopathology after exposure to different temperature regimes. Journal of Molluscan Studies (2009) 75: 9-18.

Dwek MV, Ross HA, Streets AJ et al. Helix pomatia agglutinin lectinbinding oligosaccharides of aggressive breast cancer. Int J Cancer 2001; 95:79-85.

Fuchs SY, Ougolkov AV, Spiegelman VS, Minamoto T (2005) Oncogenic beta-catenin signaling networks in colorectal cancer. Cell Cycle 4, 1522-1539.

Garefalaki ME, Triantafyllidis A, Abatzopoulos TJ, Staikou A (2010) The outcome of sperm competition is affected by behavioural and anatomical reproductive traits in a simultaneously hermaphroditic land snail. J. Evol. Biol. 23: 966-976. Garefalaki ME (2010). Invention of intraspecific differentiation of mating behavior and sperm competition in the edible snail Helix aspersa. PHD thesis. Department of Zoology, School of Biology, Aristotle University of Thessaloniki.

Guiller A. & Madec L. (2010) "Historical biogeography of the land snail Cornu aspersum: a new scenario inferred from haplotype distribution in the Western Mediterranean basin. BMC Evolutionary Biology 2010; 10-18.

Herreid CF 1977. Metabolism of land snails (Otala lacteal) during dormancy, arousal and activity. Comparative Biochemistry and Physiology, 56: 211-215.

Iglesias-de la Cruz MC, Sanz-Rodriguez F,. Zamarron A, Reyes E, E. Carrasco E, Gonzalez S, Juarranz A. A secretion of the mollusc Cryptomphalus aspersa promotes proliferation, migration and survival of keratinocytes and dermal fibroblasts in vitro International Journal of Cosmetic Science, 2012, 34, 183-189

Knudsen KA, Soler AP (2000) Cadherin mediated cell-cell interactions. Methods Mol. Biol. 137, 409-440.

Koemtzopoulos Evripidis (2007). Sexual selection in the land gastropod Helix aspersa: mating behaviour and histological invention of the sperm storing organ.

Koemtzopoulos E., Staikou A., 2007. Variation in spermathecal morphology is independent of sperm competition intensity in populations of the simultaneously hermaphroditic land snail Cornu aspersum. Zoology 110: 139-146.

Kuang S, Doran SA, Wilson RJA, Goss GG, Goldberg JI (2002) Serotonergic sensory-motor neurons mediate a behavioral response to hypoxia in pond snail embryos. Journal of Neurobiology 52:73-83.

Kumar S, Ghosh D, Biswas TK, Dutta U, Das P, Kundu S (2008) Spermatheca gland extract of snail (Telescopium telescopium) has wound healing potential: an an experimental invention in rabbits. International journal of Lower Extremity Wounds, 7,204-209. Machin J. 1966. The evaporation of water from Helix aspersa IV. Loss from the mantle of the inactive snail. Journal of Experimental Biology, 45, 269-278.

Merlin JL, Azzi S, Lignon D, Ramacci C, Zeghari N, Guillemin F (1992). MTT assays allow quick and reliable measurement of the response of human tumour cells to photodynamic therapy. Eur. J. Cancer 28A, 1452-1458.

Pons F, Koenig M, Michelot R et al. L'effet bronchorelaxant de l'hélicidine, un extrait d'Hé/ix pomatia, fait intervenir une libération de prostaglandine E2. Pathol Biol 1999;47:73-80.

Quevauviller A, Mainil J, Garcet S. Le mucus d'Hélix pomatia L. Préparation, composition, propriétés thérapeutiques et pharmacodynamiques. Rev Pathol Gen Comp 1953;653:1514-38.

Renwrantz L, Berliner U (1978) A galactose specific agglutinin, a blood-group H active polysaccharide, and a trypsin inhibitor in albumin glands and eggs of Arianta arbustorum (helicidae) Journal of Invertebrate Pathology, 31, 171-179.

Runham NW, Laryea A (1968) Studies on the maturation of the reproductive system of Agrolimax reticulates) (Pulmonata: Limacidae) Malacologia, 7, 93-108.

Skingsley DR, White AJ, Weston A (2000) Analysis of pulmonate mucus by infrared spectroscopy. Journal of Molluscan Studies, 66, 363-371.

Staikou A 1999. Shell temperature, activity and resistance to desiccation in the polymorphic land snail Cepaea vindobonensis. Journal of Molluscan Studies, 65: 171-184.

Sumner Yamada KM, Miyamoto S (1995) Integrin transmembrane signaling and cytoskeletal control. Curr. Opin. Cell Biol. 7, 681-689.

Tompa AS (1984) Land Snails (Stylommatophora). In: The Mollusca: vol.7 Reproduction, (eds. Wilbur KM ed in chief, Tompa AS, Verdonk NH, van den Biggelaar JAM). pp 47-140, London. Academic Press.

Tsoutsos D, Kakagia D, Tamparopoulos K. The efficacy of Helix aspersa Müller extract in the healing of partial thickness burns: a novel treatment for open burn management protocols. J Dermatolog Treat. 2009;20(4):219-22.

Webster L, Henderson R, Katz N, Ellis D. Characterization of confusion, an adverse event associated with intrathecal ziconotide infusion in chronic pain patients. Pain Med 2001;2:253-4.

Withers P, Pedler S, Guppy M (1997) Physiological adjustments during aestivation by the Australian land snail Rhagada tescorum (Mollusca: Pulmonata: Camaenidae). Australian Journal of Zoology, 45: 599-611.

Zouq NK, Keeble JA, Lindsay J. et al. (2009) FAK engages multiple pathways to maintain survival of fibroblasts and epithelia differential roles for paxillin and p130Cas. J. Cell Sci. 122(Pt 3), 357-367.

## Claims

1. Method for snail extract acquisition comprising at least two steps, wherein a first step for obtaining the extract consists of
(i) a non-stressful snail extract acquisition, wherein a batch of snails is selected which are brought to move by crawling on a set of surface means, yielding a quantity of pedal mucus, which is removed from said surface means, and which is followed by a further step, wherein said further step consists of
(ii) drying of the produced snail extract,
(iii) wherein said drying is followed by a subsequent resuspension step of the produced snail extract and
(iv) in that the dried snail extract is further filtered through a microporous filter having a pore size of about 0,22µm, **characterized**
- **in that** said surface means are made of inert material consisting of glass plates and
- **in that** a quantity of clear pedal mucus is yielded,
- which is placed into receptacles after said removal from said surface means.

2. Method according to the preceding claim, **characterized in that** a snail type is selected from the gastropod family of *Cornu aspersum,* an extract is acquired from the snails, wherein a fraction is extracted therefrom, wherein a said snail extract is derived from said fraction thereof.

3. Method according to one of the preceding claims, **characterized in that** there is further carried out an evaluation of the effect of all snail extracts acquired, on regenerative properties of the HaCaT human keratinocyte cell line,
wherein there is carried out an evaluation of the effect of the extracts on the HaCaT cell line by estimating the proliferation rate, the migration and wound healing potential, as well as the expression of protein-markers of cell survival (β-catenin), cell-cell adhesion (E-cadherin) and cell-substrate adhesion (integrin-β1), wherein the response of the HaCaT cell line treated with snail extracts under H₂O₂ induced oxidative stress is furthermore estimated, and wherein the potential protective or therapeutic role of bioactive substances in snail extracts is evaluated.

4. Method according to one of the preceding claims, **characterized in that** said snails are arranged to crawl or walk under optimum conditions of moisture, temperature and duration, which are defined as a high humidity and room temperature of approximately 18° to 22°, particularly of about 20°C, on said surface means for about 2 hours, particularly wherein said receptacles consist of petri dishes of glass.

5. Method according to the preceding claim, **characterized in that** the Dry extract is obtained by placing the snail extracts into a set of inert substantially smooth plates, particularly glass petri dishes, into a chamber at approximately 40°C a certain duration corresponding thereto, notably of about 24h; more particularly wherein the dried snail extract is resuspended in aqueous solution, notably with a pH of about 7,4, and filtered through said microporous filter having said pore size.

6. Method according to one of the claims 1 and 2, or resp. following ones, **characterized in that** said snail extracts are acquired essentially at any of the three sampling seasons, which are determined either as
(a) during a period of reproductive activity, essentially circumscribed between the months of September to November in the area of origin being the Mediterranean, resp.
(b) during a period of hibernation between the months of December to February and/or
(c) during a period of activity between the months of March to May.

7. Method according to the preceding claim, **characterized in that** said snail extracts are acquired at the optimum sampling season for snail extracts being any of both sampling seasons mainly during said period of reproductive activity of snails (a), and also during said period of activity (c);
possibly **in that** said snail extracts are acquired when snail mucus is produced by snails during activity, either reproductive or not, as for defense and locomotion of gastropods that are exposed to the external environment.

8. Method according to one of the claims 3 to 6, **characterized in that** a bioactive potential of snail extracts acquired of *Cornu aspersum* is identified at all levels of said evaluation process, wherein this extract induces significantly the proliferation rate, the migration and wound healing potential, as well as the expression of all protein-markers tested.

9. Method according to any one of the preceding claims, **characterized in that** it is started from a snail extract of *Cornu aspersum* of natural origin.

10. Method according to any one of the claims 1 to 8, **characterized in that** it is started from a snail extract of *Cornu aspersum* from a cultured origin, particularly applied in a large scale such as in cooperation with snail farms.

11. Snail extract obtained according to a method as defined in any one of the claims 1 to 10 for use in the treatment of a disease relating to skin ageing, skin exposure to various damaging environmental factors, or skin burns.

12. Cosmetic use of the snail extract obtained according to a method as defined in any one of the claims 1 to 10.

13. Pharmaceutical product comprising the snail extract obtained according to a method as defined in any one of the claims 1 to 10.

14. Snail extract for use according to claim 11, wherein the secretion of snails is used in the regeneration of burnt skin and in the management of open wounds.

## Patentansprüche

1. Verfahren zur Gewinnung von Schneckenextrakt die wenigstens zwei Schritte umfasst, wobei der erste Schritt zur Extrakt Gewinnung Folgendes umfasst:
i) eine stressfreie Extrakt Gewinnung, bei der eine Charge Schnecken dazu gebracht wird, auf einem Satz von Oberflächenmedien zu kriechen, wobei eine bestimmte Menge an Kriechschleim gewonnen wird, der den genannten Flächen entnommen wird, diesem Schritt folgt weiterer Schritt, der aus folgenden Einzelschritten besteht
ii) Trocknen des Schneckenextrakts,
iii) wobei die genannte Trocknung folgender Schritt ist einer Resuspendierung des hergestellten Schneckenextrakts und
iv) eine Filterung des getrockneten Schneckenextrakts durch einen mikroporösen Filter mit einer Porengröße von etwa 0,22 µm, **dadurch gekennzeichnet**
- **dass** genannte Oberflächen aus inertem Material bestehen, das aus Glasplatten besteht, und
- **dass** eine bestimmte Menge an klarem Kriechschleim abgegeben wird,
- wobei der Schleim nach der Entnahme von den genannten Oberflächen in Behältnisse gelegt wird.

2. Verfahren nach dem vorhergehenden Anspruch, **dadurch gekennzeichnet dass** eine Schneckenart aus der Schneckenfamilie der *Cornu aspersum* ausgewählt wird, und ein Extrakt aus die Schnecken gewonnen wird, wobei eine Fraktion davon gewonnen wird, wobei das genannte Schneckenextrakt aus dieser Fraktion gewonnen wird.

3. Verfahren nach einem der vorhergehenden Ansprüche, **dadurch gekennzeichnet dass** darüber hinausgehend eine Bewertung der Wirkung der gewonnenen Schneckenextrakte auf die regenerativen Eigenschaften der HaCaT humanen Keratinozytenzelllinie vorgenommen wird,
wobei die Wirkung des Extrakts auf die HaCaT-Zellinie durch Schätzung der Zellproliferationsrate, des Migrations- und des Wundheilungspotenzials sowie der Expression der Proteinmarker für das Überleben von Zellen (β-Catenin), der Zell-Zell-Adhäsion (E-Cadherin) und der Zell-Substrat-Adhäsion (Integrin-Bt) bewertet wird und die Reaktion der mit Schneckenextrakten behandelten HaCaT-Zelllinie unter H₂0₂-induziertem oxidativem Stress weiter abgeschätzt wird, und wobei die potenzielle schützende oder therapeutische Rolle der bioaktiven Substanzen in Schneckenextrakten bewertet wird.

4. Verfahren nach einem der vorhergehenden Ansprüche, **dadurch gekennzeichnet dass** die Schnecken so positioniert werden, dass sie unter optimalen Feuchtigkeits-, Temperatur- und Zeitbedingungen definiert als eine hohe Luftfeuchtigkeit und Raumtemperatur etwa 18° bis 22°, besonders etwa 20°C, auf genannten Oberflächen kriechen, etwa 2 Stunden lang, insbesondere wobei die genannten Behältnisse gläserne Petrischalen sind.

5. Verfahren nach dem vorhergehenden Anspruch, **dadurch gekennzeichnet dass** das Trockenextrakt gewonnen wird, indem das Schneckenextrakt für eine bestimmte damit einsprechende Dauer, bemerkenswert etwa 24 Stunden bei etwa 40°C in eine Set inerte, im Wesentlichen glatte Behältnisse, besonders Petrischalen aus Glas, in eine Kammer eingebracht wird;
insbesondere wobei der getrocknete Schneckenextrakt in einer wässrigen Lösung, bemerkenswert mit einem pH-Wert von etwa 7,4, resuspendiert und durch genannte Mikrofilter mit genannter Porengröße filtriert wird.

6. Verfahren nach einem der Ansprüche 1 oder 2, oder bzw. einem der folgenden Ansprüche, **dadurch gekennzeichnet dass** das Schneckenextrakt im Wesentlichen zu einer der drei folgenden Probenjahreszeiten gewonnen wird:
a) während der Fortpflanzungszeit, die im Herkunftsgebiet im Mittelmeerraum die Monate September bis November umfasst, bzw.
b) während der Winterruhe in den Monaten Dezember bis Februar und/oder
c) während der Aktivitätsperiode von März bis Mai.

7. Verfahren nach dem vorhergehenden Patentanspruch, **dadurch gekennzeichnet dass** die Schneckenextrakte zur optimalen Probenjahreszeit für Schneckenextrakte gewonnen wirden, also eine der beiden Probenjahreszeiten, im wesentlich während die Fortpflanzungszeit der Schnecken (a), und auch während die Aktivitätsperiode (c);
möglicherweise wobei die genannten Extrakte gewonnen wirden wenn die Schnecken während der fruchtbaren oder der unfruchtbaren Periode Schneckenschleim produzieren, sei es als Abwehrmechanismus und aus Gründen der Fortbewegung von der Außenumgebung ausgesetzten Schnecken.

8. Verfahren nach den Ansprüchen 3 bis 6, **dadurch gekennzeichnet dass** das bioaktive Potenzial von Schneckenextrakte aus *Cornu aspersum* in allen Stufen des genannten Evaluierungsverfahrens identifiziert wird, wobei dieses Extrakt die Proliferationsrate, das Migrations- und Wundheilungspotenzial sowie die Expression aller getesteten Proteinmarker signifikant steigert.

9. Verfahren nach einem beliebigen vorhergehenden Anspruch, **dadurch gekennzeichnet dass** es von einem Schneckenextrakt aus *Cornu aspersum* natürlichen Ursprungs ausgeht.

10. Verfahren nach einem der Ansprüche 1 bis 8, **dadurch gekennzeichnet dass** es von einem Schneckenextrakt aus *Cornu aspersum* kultivierten Ursprungs ausgeht, besonders in großem Maßstab, wie in Zusammenarbeit mit einer Schneckenfarm angewandt wird.

11. Schneckenextrakt, gewonnen nach einem Verfahren wie definiert in einem der Ansprüche 1 bis 10, zur Verwendung bei der Behandlung einer Krankheit, die mit der Hautalterung, der Exposition der Haut gegenüber verschiedenen schädlichen Umweltfaktoren oder Hautverbrennungen zusammenhängt.

12. Kosmetische Verwendung des Schneckenextrakts, gewonnen nach einem Verfahren wie definiert in einem der Ansprüche 1 bis 10.

13. Pharmazeutisches Produkt, das Schneckenextrakt gewonnen nach einem Verfahren wie definiert in einem der Ansprüche 1 bis 10 enthält.

14. Schneckenextrakt zur Verwendung nach Anspruch 11, wobei das Schneckensekret zur Regeneration verbrannter Haut und zur Behandlung offener Wunden verwendet wird.

## Revendications

1. Procédé de collecte d'extrait d'escargots comprenant au moins deux étapes, la première étape pour obtenir l'extrait consistant à :
(i) collecter un extrait d'escargots de manière non stressante, dans lequel un lot d'escargots est sélectionné qui sont amenés à se déplacer en rampant sur un ensemble de moyens de surface, produisant une quantité de mucus de pédalage, qui est retiré desdits moyens de surface, et qui est suivi d'une autre étape, ladite étape suivante consistant à
(ii) sécher l'extrait d'escargots produit,
(iii) ledit séchage étant suivi d'une étape de remise en suspension ultérieure de l'extrait d'escargots produit et
(iv) l'extrait d'escargots séché étant filtré ensuite à travers un filtre microporeux ayant une taille de pore d'environ 0,22 µm, **caractérisé en ce que**
- lesdits moyens de surface sont réalisés en un matériau inerte consistant en plaques de verre et
- une certaine quantité de mucus de pédalage clair est produit,
- qui est placée dans des réceptacles après le retrait précité à partir desdits moyens de surface.

2. Procédé selon la revendication précédente, **caractérisé en ce qu'**un type d'escargots est sélectionné à partir de la famille des gastéropodes *Cornu aspersum,* un extrait étant collecté à partir des escargots, dont une fraction est extraite, ledit extrait d'escargots étant dérivé de ladite fraction de celui-ci.

3. Procédé selon l'une des revendications précédentes, **caractérisé en ce qu'**on effectue ensuite une évaluation de l'effet de tous les extraits d'escargots collectés sur les propriétés régénératrices de la lignée cellulaire HaCaT de kératinocytes humains, dans lequel on effectue une évaluation de l'effet des extraits sur la lignée cellulaire HaCaT en estimant le taux de prolifération, le potentiel de migration et de cicatrisation, ainsi que l'expression de marqueurs protéiques de la survie cellulaire (β-caténine), de l'adhésion cellulaire (cadhérine E) et de l'adhésion des cellules au substrat (integrin-β1), dans lequel on estime ensuite la réponse de la lignée cellulaire HaCaT traitée avec des extraits d'escargots sous stress oxydatif induit par H₂O₂ et dans lequel le rôle protecteur ou thérapeutique potentiel des substances bioactives dans les extraits d'escargots est évalué.

4. Procédé selon l'une des revendications précédentes **caractérisé en ce que** lesdits escargots sont amenés à ramper ou se balader dans des conditions optimales d'humidité, de température et de durée, qui sont définies comme une humidité élevée et une température ambiante d'environ 18°C à 22°C, en particulier d'environ 20°C, sur lesdits moyens de surface pendant environ 2 heures, particulièrement dans lequel lesdits réceptacles consistent en boîtes de Pétri en verre.

5. Procédé selon la revendication précédente, **caractérisé en ce que** l'extrait sec est obtenu en plaçant les extraits d'escargots dans un ensemble de plaques inertes sensiblement lisses, en particulier des boîtes de Pétri en verre, dans une pièce à environ 40°C, une certaine durée correspondant à cela d'environ 24h ;
plus particulièrement dans lequel l'extrait d'escargots séché est remis en suspension dans une solution aqueuse, notamment avec un pH d'environ 7,4 , et filtré à travers un filtre microporeux avec des pores de la taille susmentionnée.

6. Procédé selon l'une des revendications 1 et 2, ou respectivement les suivantes, **caractérisé en ce que** lesdits extraits d'escargots sont collectés principalement au couèrs d'une des trois saisons d'échantillonnage, qui sont déterminées chacune comme suit
(a) pendant une période d'activité de reproduction, essentiellement comprise entre les mois de septembre et novembre dans la zone d'origine, c'est-à-dire la Méditerranée, respectivement
(b) pendant une période d'hibernation entre les mois de décembre à février et/ou
(c) pendant une période d'activité entre les mois de mars à mai.

7. Procédé selon la revendication précédente, **caractérisé en ce que** l'extrait d'escargots est collecté pendant la saison d'échantillonnage optimale, soit l'une des deux saisons d'échantillonnage, principalement pendant la période d'activité de reproduction des escargots (a), ainsi que pendant la période d'activité (c) ; éventuellement **en ce que** lesdits extraits d'escargots étant collectés lorsque le mucus d'escargot est produit par les escargots pendant l'activité, qu'elle soit de reproduction ou non comme pour la défense et la locomotion de gastéropodes qui sont exposés à l'environnement externe.

8. Procédé selon l'une des revendications 3 à 6, **caractérisé en ce qu'**un potentiel bioactif issu d'extraits d'escargots collectés de la famille *Cornu aspersum* est identifié à tous les niveaux du processus d'évaluation, cet extrait influant significativement sur le taux de prolifération, le potentiel de migration et de cicatrisation, ainsi que sur l'expression de tous les marqueurs protéiques testés.

9. Procédé selon l'une quelconque des revendications précédentes, **caractérisé en ce qu'**on part d'un extrait d'escargots de la famille des *Cornu aspersum* d'origine naturelle.

10. Procédé selon l'une quelconque des revendications 1 à 8, **caractérisé en ce qu'**on part d'un extrait d'escargots de la famille des *Cornu aspersum* d'origine cultivée, ce procédé étant particulièrement appliqué à grande échelle, comme en coopération avec des élevages d'escargots.

11. Extrait d'escargots obtenu selon un procédé tel que défini dans l'une quelconque des revendications 1 à 10 pour une utilisation dans le traitement des maladies liées au vieillissement de la peau, à l'exposition de la peau à divers facteurs environnementaux dommageables ou aux brûlures de la peau.

12. Utilisation cosmétique de l'extrait d'escargots obtenu selon un procédé tel que défini dans l'une quelconque des revendications 1 à 10.

13. Produit pharmaceutique comprenant l'extrait d'escargots obtenu selon un procédé tel que défini dans l'une quelconque des revendications 1 à 10.

14. Extrait d'escargots à utiliser selon la revendication 11, dans lequel la sécrétion d'escargots est utilisée pour la régénération de peau brûlée et pour le traitement de plaies ouvertes.
